# EUROPÄISCHE PATENTANMELDUNG

(11) **EP 1 671 626 A1**
(43) Veröffentlichungstag der Anmeldung: **21.06.2006**
(21) Anmeldenummer: 05027489.3
(22) Anmeldetag: 15.12.2005
(51) Int. Cl.: A61K 9/70, A61K 31/365, A61K 31/565

(54) **Transdermales Pflaster mit Progesteron A-spezifischen Liganden (PRASL) als Wirkstoff**

(30) Priorität: 20.12.2004 DE 102004062182
(71) Anmelder: Schering Aktiengesellschaft, 13342 Berlin (DE)
(72) Erfinder: Prodhaisky, Hans-Peter, 13156 Berlin (DE); Bracht, Stefan, 16548 Glienicke Nordbahn (DE)
(74) Vertreter: Cramer, Eva-Maria

(57) **Zusammenfassung**

Die Erfindung betrifft ein transdermales Pflaster mit Progesteron A-spezifische Liganden (PRASL) als Wirkstoff, bestehend aus einer Rückschicht, mindestens einer daran haftenden, wirkstoffhaltigen Klebschicht auf Basis eines Silikonpolymers, eines Polyisobutylenpolymers (PIB), eines Polyacrylatpolymers oder eines Styrolblockcopolymers mit Butadien oder Isopren (SBS oder SIS), sowie eines entfernbaren Schutzfilmes. Das erfindungsgemäße transdermale therapeutische Pflaster, PRASL enthaltend gegebenenfalls auch in Kombination mit Estrogenen eignet sich zur Hormonersatztherapie und zur Fertilitätslcontrolle.

## Beschreibung

### Technisches Gebiet

Die Erfindung betrifft ein transdermales Pflaster mit Progesteron A-spezifische Liganden (PRASL) als Wirkstoff.

Das erfindungsgemäße transdermale therapeutische Pflaster, PRASL enthaltend gegebenenfalls auch in Kombination mit Estrogenen eignet sich zur Hormonersatztherapie und zur Fertilitätskontrolle.

### Stand der Technik

Aus der Patentliteratur, WO 03/075915 A1 ist eine die transdermale Applikation von PRASL bekannt. Hierbei stellt diese als
- ein allgemein bekanntes transdermalen Pflasters, welches optional einen Penetrationsenhancer enthält oder
- eine Emulsion, eine Salbe, eine Cream oder ein Gel dar.

Die Mehrzahl der bekannten transdermalen Pfalster sind passive Matrixsysteme, bestehend aus einer weitgehenden wasserdampfundurchlässigen und für den Wirkstoff undurchlässigen Rückschicht, einer wirkstoffhaltigen Kleberschicht und einer wiederablösbaren Schutzschicht. Bei den bisher bekannten passive Matrixsystemen liegt der Wirkstoff dabei vollständig gelöst in der Kleberschicht vor.

Diese Form der Zubereitung kann aber auch nachteilig sein.

Die Freisetzung von gelöstem Wirkstoffe aus der Pflastermatrix verläuft nichtlinear sondern nähert sich, entsprechend des 2. Fickschen Gesetzes. asymptotisch einem Maximalwert an. Dieser Maximalwert ist u.a. durch die Sättigungskonzentration des Wirkstoffes in der Matrix begrenzt. Das heißt mit vorschreitender Applikationsdauer vermindert sich, die vom Pflaster pro Zeiteinheit and die Haut freigegebene Wirkstoffmenge. Insbesondere für eine hormonhaltige Arzneiform, die über einen längeren Zeitraum von mehreren Tagen angewandt werden soll, wäre aber eine lineare Freigabe des Wirkstoffes über den gesamten Applikationszeitraum sehr wünschenswert, da dies der physiologischen Sekretion der Hormone sehr viel mehr entspricht. Eine derartige Freisetzungskinetik ist mit den bisher bekannten transdermalen Pflastern nicht realisierbar.

Die Patentschrift WO 03/075915 A1 schlägt im Weiteren die Nutzung einer Emulsion, einer Salbe, einer Cream oder eines Gels vor. Die Nutzung einer Emulsion einer Salbe, einer Cream oder eines Gels als Applikationsform einer PRASL-haltigen Arzneiform erscheint allerdings aus verschiedenen Gründen wenig geeignet. So muss die offene Anwendung eines hochpotenten Arzneistoffes-(PRASL löst pharmakologische Effekte bereits in Tagesdosen ab 30 µg aus) als problematisch betrachtet werden. Transdermale Gele werden in der Regel großflächig auf die Haut aufgetragen (100-200 cm²). Es ist bekannt. dass der größte Teil des Arzneistoffes für eine längere Zeit auf der Hautoberfläche verbleibt und erst über den Zeitraum von mehreren Stunden vollständig in tiefere Hautschichten eindringt und dann z.T. resorbiert wird. Damit geht aber eine nicht unerhebliche Gefahr einer Partnerkontamination aus. Durch Hautkontakt können größere Mengen des Wirkstoffes übertragen werden und somit zu einer unkontrollierten Behandlung einer unbeteiligten Person führen. Darüber hinaus können Teile des offen auf der Hautoberfläche applizierten Wirkstoffes z.B. während des Duschens in die Kanalisation gelangen und so eine Umweltbelastung verursachen.

Weiterhin ist bekannt, dass das Molekulargewicht eines Arzneistoffes eine limitierende Größe für seine transdermale Verfügbarkeit darstellt. Wirkstoffe wie PRASL mit einem Molekulargewicht von MW ≥ 500 Da gelten als gering hautgängig.

Daher muss man davon ausgehen, dass bei den konventionellen Pflastern mit PRASL ein hoher Anteil des Wirkstoffes eingesetzt werden muss, um einen wirksamen Plasmaspiegel zu erhalten.

### Darstellung der Erfindung

Der vorliegenden Erfindung liegt somit die Aufgabe zugrunde, ein transdermales Pflaster bereit zu stellen, welches einen pharmakologisch wirksame Plasmaspiegel von PRASL (entsprechend einer transdermalen Verabreichung von ca. 30-50 µg/d) realisiert.

Um das Risiko des Auftretens von Wirkstoff-Hilfsstoff Inkompatibilitäten sowie von irritativen Hautreaktionen zu vermeiden, sollte diese Pflaster möglichst keine penetrationsverstärkende Hilfsstoffzusätze enthalten.

Ferner sollte ein transdermales Pflaster mit dem Wirkstoff PRASL zur Verfügung gestellt werden, welches einen möglichst großen Anteil des verarbeiteten Wirkstoffes freisetzt und demzufolge ein Pflaster mit einem geringen prozentualen Anteil des Wirkstoffes in der Arzneiform realisiert.

Um mittels einer Reduktion des Dosierintervalles eine Erhöhung der Akzeptanz durch die Patienten zu erreichen, soll weiterhin ein solches transdermales Pflaster mit PRASL gefunden werden, welches einen linearen Wirkstofftransport über einen Zeitraum von mehreren Tagen ermöglicht,

Erfindungsgemäß wird die Aufgabe durch ein transdermales Pflaster, den Wirkstoff der allgemeinen Formel - 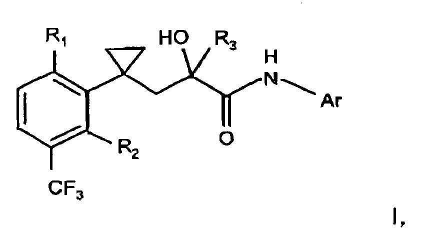 enthaltend , wobei R₁ und R₂ unabhängig voneinander H oder F, R₃ = CH₃ oder CF₃ und AR = 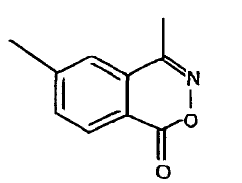 oder 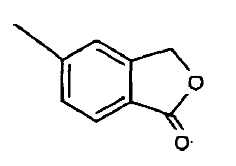 oder pharmazeutisch geeignete Derivate oder Abkömmlinge hiervon sind (Progesteron A-spezifische Liganden, PRASL) gelöst, wobei das transdermale Pflaster aus einer Rückschicht, mindestens einer daran haftenden, wirkstoffhaltigen Klebschicht auf Basis eines Silikonpolymers, eines Polyisobutylenpolymers (PIB), eines Polyacrylatpolymers oder eines Styrolblockcopolymers mit Butadien oder Isopren (SBS oder SIS), wobei der Wirkstoff in einer Konzentration von 0.1-10, bezogen auf das Gesamtgewicht der Klebematrix enthalten ist, sowie eines entfernbaren Schutzfilmes besteht.

Ferner kann erfindungsgemäß der Wirkstoff in einer Konzentration von 0,1-5%, bezogen auf das Gesamtgewicht der Klebermatrix vorzugsweise in der Konzentrationen von 0,1-2% bezogen auf das Gesamtgewicht der Klebermatrix enthalten sein.

Auch kann beim erfindungsgemäßen transdermalen Pflaster die Löslichkeit des Wirkstoffes in der Klebschicht von 0,1-5% , vorzugsweise von 0,5-2% definiert sein.

Beim erfindungsgemäßen transdermalen Pflaster kann der Wirkstoff in weniger als 50% ungelöst in der Matrix eingebettet sein.

Dabei kann erfindungsgemäß der ungelöste Wirkstoff als gleichmäßige Dispersion von MiKropartikeln oder Mikrotropfen, vorzugsweise als Nanopartikel oder Nanotropfen vorliegen.

Besonders bevorzugt liegt der Wirkstoff in amorpher Form in der Matrix vor.

Die erfindungsgemäße amorphe Wirkstoffdispersion in der Klebermatrix bietet gegenüber den bisher bekannten, kristallinen Dispersionen bedeutende Vorteile:
- die amorphe Dispersion weist eine besonders große Grenzfläche des ungelösten Wirkstoffes gegenüber der Matrix auf, wodurch das Nachlösen von Wirkstoff für die Freigabe aus der Matrix erleichtert wird;
- im amorphen Zustand müssen für das Nachlösen von Wirkstoff während der Applikation im Gegensatz zu Kristallen keine Gitterenergien aufgebracht werden, so dass dieser Auflösungsprozeß sich gegenüber der Freisetzung von Wirkstoff aus der Matrix nicht als limitierend für die Abgaberate darstellt;
- die amorphe Wirkstoffdispersion bietet ein homogenes optisches Bild; bei einer kristallinen Dispersion kann der Anwender das Auftreten von Spots beobachten, was ihn auf eine mindere Qualität des transdermalen Pflasters schließen lässt und so zu Akzeptanzproblemen führen kann.

Bei dem erfindungsgemäßen transdermalen Pflaster kann/ können auch die in der wirkstoffhaltigen Matrix enthaltenden Kristallisationsinhibitoren aus der Gruppe ausgewählt sein, die N-Vinyllactam-Polymere wie N-Vinyl-1-Azacycloheptan-2-on-Homapolymer und N-Vinyl-piperidin-2-on-Homopolymer und insbesondere Polymere des Vinylpyrrolidons, wie Polyvidon (Kollidon TM), oder Co-Polymere des Vinylpyrrolidons mit Vinylacetat (Copovidone) umfasst.

Der Kristallisationsinhibitor kann ein Copovidon aus 6 Teilen Vinylpyrrolidon und 4 Teilen Vinylacetat (Kollidon TM VA 64) sein.

Bei dem erfindungsgemäßen transdermalen Pflaster kann die Klebschicht einen Klebestoff auf Silikonbasis enthalten, der sich durch einen hohen Anteil an Polymer gegenüber dem Harz auszeichnen, vorzugsweise aminkompatible Klebstoffe mit einem Masseverhältnis Polymer zu Harz größer oder gleich 40% zu 60%.

Ferner kann/können bei dem erfindungsgemäßen transdermalen Pflaster die Klebschicht Klebestoffe auf Polyisobutylenbasis enthalten.

Auch kann/können das erfindungsgemäße transdermale Pflaster zusätzlich ein Estrogen enthalten , das aus der Gruppe ausgewählt ist. die 17-beta-Estradiol, Ethinylestradiol, Estradiolvalerat, Estradiolcypionat, Estradialacetate und Estradiolbenzuate umfasst.

Ferner kann bei dem erfindungsgemäßen transdermalen Pflaster innerhalb eines 7-tägigen Anwendungszyklus mehr als 30%, vorzugsweise mehr als 50% der Beladung seines Wirkstoffes freigesetzt werden.

Ferner kann das erfindungsgemäße transdermalen Pflaster hergestellt werden, indem der entsprechende Wirkstoff in einer Kombination aus mindestens zwei Prozesslösemittein aufgenommen wird, von denen eines ein geringes Lösungsvermögen für den Wirkstoff und ein weiteres ein hohes Lösungsvermögen für den Wirkstoff besitzt, wobei letzteres nach Vermengung mit der Klebermatrix durch Trocknungsprozesse zuerst aus dem Ansatz entfernt wird.

Dabei kann das Lösungsmittel mit geringem Lösungsvermögen für den Wirkstoff 1,4 Dioxan und das Lösungsmittel mit hohem Lösungsvermögen Heptan sein.

Folgende erfindungsgemäße in Detailschritten aufgeführte Formulierungsstrategie kennzeichnet die Herstellung der PRASL-haltigen Pflaster:
1. die Auswahl von Klebermatrizes mit optimierten Lösungseigenschaften für PRASL,
2. eine geeignete Methode der Herstellung und
3. durch die Auswahl von geeigneten Stabilisierungszusätzen für die Matrix aus.

### 1. Auswahl der Klebermatrizes

Es erfolgt die Auswahl von geeigneten Klebermatrizes aufgrund ihrer Lösungseigenschaften für PRASL. Geeignete Klebermatrizes im Sinne der vorliegenden Erfindung sind solche, in denen die Löslichkeit für PRASL zwischen 0,1-5% liegt. Als besonders geeignet haben sich Klebermatrizes herausgestellt, in denen die Löslichkeit von PRASL zwischen 0,1-2% liegt.

Als medizinisch akzeptable Kleber dieser Klebermatrix können beispielsweise Silikon, Polyacrylat-, oder Polyisobutylen-Kleber eingesetzt werden. Darüber hinaus sind aber auch Polyurethane, Block-Copolymere auf Styrenbasis und weitere organische Polymere einsetzbar.

Besonders bevorzugt im Sinne der vorliegenden Erfindung sind zur medizinischen Anwendung geeignete Silikonkleber, die einen möglichst hohen Anteil des Polymers gegenüber dem Harz aufweisen und hierbei insbesondere solche, die aminkompatibel sind, wie z.B. die Bio PSA® Serie von Dow Corning sowie polyisbutylenhaltige Kleberzubereitungen wie beispielsweise eine Zubereitung aus folgenden Komponenten, die auf bekannte Art und Weise hergestellt wird:

Beispiel für eine erfindungsgemäße, polyisobutylenhaltige Klebermatrix.

| Komponente | Gewichtsanteil in getrockneter Klebermatrix |
|---|---|
| PRASL | 1 |
| Oppanol B100 | 10 |
| Oppanol B 12 SFN | 52,2 |
| Indopol H 2100 | 35 |

### 2. Methode zur Herstellung zur Einarbeitung von PRASL in die Matrix

PRASL wird während des Herstellprozesses in einer Kombination aus mindestens zwei Prozesslösemitteln aufgenommen , von denen eines ein geringes Lösungsvermögen für die Wirkstoffe (z.B. Heptan) und ein weiteres ein hohes Lösungsvermögen für die Wirkstoffe (z.B. 1,4 Dioxan) besitzt.

Dabei kann eines der beiden Lösemittel auch eine Komponente der flüchtigen Bestandteile der Klebermatrix sein. Nach Vermengung der PRASL-Lösung mit der Klebermatrix wird durch Trocknungsprozesse zuerst das Lösemittel aus dem Ansatz entfernt, das gute Lösungseigenschaften für PRASL besitzt.

Überraschenderweise kommt es während der anschließenden Filmbildung zum spontanen Ausfallen eines Teil des Wirkstoffes in der Form einer amorphen Dispersion, deren Partikel überwiegend die Größe von Nanopartikeln aufweisen. Das Ausfällen dieser amorphen Dispersion setzt den Einsatz von Klebermatrizes mit den oben beschriebenen Lösungseigenschaften für PRASL voraus.

Ist das Lösevermögen der verwendeten Klebermatrix für PRASL größer als 2-5%, wie z.B. im Fall der Acrylat-Klebermatrix DuroTak 387-2287, bleibt die gesamte Wirkstoffmenge auch nach der Filmbildung gelöst und die Vorteile einer erfindungsgemäßen amorphen Wirkstoffdispersion können nicht genutzt werden.

### 3. Auswahl von geeigneten Stabilisierungszusätzen für die Matrix

Zur Stabilisierung dieser amorphen Dispersion des Wirkstoffes in der Klebermatrix können Kristallisationsinhibitoren zugesetzt werden. Hierbei handelt es sich um, dem Fachmann bekannte, pharmazeutische Hilfsstoffe, die als Komplexbildner geeignet sind, beispielsweise feste Lösungen mit Wirkstoffen zu bilden. die Grenzflächenlöslichkeit für den Wirkstoff erhöhen und die Neigung des Wirkstoffes zur Rekristallisation nach Entfernen eines Prozesslösungsmittels oder Senkung der Temperatur herabsetzen. Der Zusatz von Kristallisationsinhibitoren ermöglicht es, die amorphe Dispersion des Wirkstoffes in der Klebermatrix zu stabilisieren, in dem eine weitere Fällung des gelösten Wirkstoffanteils verhindert wird und darüber hinaus eine Umwandlung der amorphen Partikel in kristalline Partikel blockiert wird.

### Ausführungsbeispiel

Die Erfindung wir an nachfolgendem Beispiel näher erläutert Matrixzusammensetzung von erfindungsgemäßen transdermalen Pflastern

| Beispiel | Komponenten | Gewichtsanteil in getrockneter Klebermatrix in [%] | Kommentar |
|---|---|---|---|
| 1 | -(R)-3-{1-[2-Fluor-5-(trifluormethyl) phenyl] cyclopropyl}-2-hydroxy -N-(phthalid - 5-yl) - 2-(trifluoromethyl) propanamid | 0,25 | Herstellung einer erfindungsgemäßen amorphen Dispersion des Wirkstoffes in der Klebermatrix ist nicht möglich, da der Wirkstoff auch nach Filmbildung vollständig gelöst bleibt |
| | -BioPSA 4302 | 99,75 | |
| 2 | -(R)-3-{1-[2-Fluor-5-(trifluormethyl) phenyl] cyclopropyl}-2-hydroxy -N-(phthalid - 5-yl) - 2-(trifluoromethyl) propanamid | 0,5 | Herstellung einer erfindungsgemäßen amorphen Dispersion des Wirkstoffes in der Klebermatrix ist möglich - |
| | -BioPSA 4302 | 99,5 | |
| 4 | -(R)-3-{1-[2-Fluor-5-(trifluormethyl) phenyl] cyclopropyl}-2-hydroxy -N-(phthalid - 5-yl) - 2-(trifluoromethyl) propanamid | 0,75 | Herstellung einer erfindungsgemäßen amorphen Dispersion des Wirkstoffes in der Klebermatrix ist möglich |
| | -BioPSA 4302 | 99,25 | |
| 4 | -(R)-3-{1-[2-Fluor-5-(trifluormethyl) phenyl] cyclopropyl}-2-hydroxy -N-(phthalid - 5-yl) - 2-(trifluoromethyl) propanamid | 1 | Herstellung einer erfindungsgemäßen amorphen Dispersion des Wirkstoffes in der Klebermatrix ist möglich |
| | -BioPSA 4302 | 99 | |
| 5 | -(R)-3-{1-[2-Fluor-5-(trifluormethyl) phenyl] cyclopropyl}-2-hydroxy -N-(phthalid - 5-yl) - 2-(trifluoromethyl) propanamid | 1 | Herstellung einer erfindungsgemäßen amorphen Dispersion des Wirkstoffes in der Klebermatrix ist möglich |
| | -Oppanol B100 | 10 | |
| | -Oppanol B 12 SFN | 52,2 | |
| | - Indopol H 2100 | 35 | |
| 6 | -(R)-3-{1-[2-Fluor-5-(trifluormethyl) phenyl] cyclopropyl}-2-hydroxy -N-(phthalid - 5-yl) - 2-(trifluoromethyl) propanamid | 1 | Herstellung einer erfindungsgemäßen amorphen Dispersion des Wirkstoffes in der Klebermatrix ist nicht möglich, da Wirkstoff auch nach der Filmbildung vollständig gelöst bleibt |
| | - DuroTak 387-2287 | 99 | |
| 7 | -(R)-3-{1-[2-Fluor-5-(trifluormethyl) phenyl] cyclupropyl}-2-hydroxy -N-(phthalid - 5-yl) - 2-(trifluoromethyl) propanamid | 2 | Herstellung einer erfindungsgemäßen amorphen Dispersion des Wirkstoffes in der Klebermatrix ist nicht möglich, da Wirkstoff auch nach der Filmbildung voll- |
| | - DuroTak 387-2287 | 98 | ständig gelöst bleibt |
| 8 | -(R)-3-{1-[2-Fluor-5-(trifluormethyl) phenyl] cyclopropyl}-2-hydroxy -N-(phthalid - 5-yl) - 2-(trifluoromethyl) propanamid | 3 | Herstellung einer erfindungsgemäßen amorphen Dispersion des Wirkstoffes in der Klebermatrix ist nicht möglich, da Wirkstoff auch nach der Filmbildung voll- |
| | - DuroTak 387-2287 | 97 | ständig gelöst bleibt |
| 9 | -(R)-3-{1-[2-Fluor-5-(trifluormethyl) phenyl] cyclopropyl}-2-hydroxy -N-(phthalid - 5-yl) - 2-(trifluoromethyl) propanamid | 4 | Herstellung einer erfindungsgemäßen amorphen Dispersion des Wirkstoffes in der Klebermatrix ist nicht möglich, da Wirkstoff auch nach der Filmbildung voll- |
| | - DuroTak 387-2287 | 96 | ständig gelöst bleibt |
| 10 | -(R)-3-{1-[2-Fluor-5-(trifluormethyl) phenyl] cyclopropyl}-2-hydroxy -N-(phthalid - 5-yl) - 2-(trifluoromethyl) propanamid | 5 | Herstellung einer erfindungsgemäßen amorphen Dispersion des Wirkstoffes in der Klebermatrix ist nicht möglich, da Wirkstoff auch nach der Filmbildung voll- |
| | - DuroTak 387-2287 | 95 | ständig gelöst bleibt |

### Nachfolgend wird Beispiel 4 näher erläutert.

Eine 10%ige Lösung des erfindungsgemäßen Wirkstoffes (R)-3-{1-[2-Fluor-5-(trifluormethyl) phenyl]cyclopropyl}-2-hydroxy -N- (phthalid - 5-yl) - 2-(trifluoromethyl) propanamid

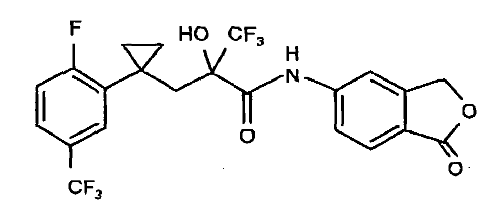

in Dioxan wird in einem geeigneten Ansatzbehältnis vorgelegt. Ein Aliquot einer geeigneten Klebermatrix (z.B. BioPSA ® 4302, Dow Corning) wird hinzugefügt, so dass sich eine 1%ige Mischung, bezogen auf den Feststoffgehalt der Matrix, ergibt.

Die Matrix wird auf eine bekannte Art und Weise homogenisiert. Der Film wird auf einer wiederabziehbaren Schutzschicht (z.B. Scotchpack 9742®, 3M) ausgestrichen und entsprechend der pharmazeutischen Regeln getrocknet. Beim Abdampfen der Lösemittel kommt es zur teilweisen Ausfällung des Wirkstoffes in der Form von gleichmäßig dispergierten Nanopartikein in der Klebstoffmatrix. Abschließend erfolgt Kaschieren mittels einer weitgehend wasserdampf-undurchlässigen Rückschicht (z.B. Hostaphan RN 15 MED®, Mitsubishi) und das Vereinzeln der Pflaster.

Nachfolgend sind beispielhaft die Ergebnisse der *in vitro* Liberation für 6 dieser erfindungsgemäßen Pflaster über den Zeitzraum von 24 h dargestellt.

**Tabelle 1 Prozentuale Freisetzung von (R)-3-{1-[2-Fluor-5-(trifluormethyl) phenyl] cyclopropyl}-2-hydroxy -N-(phthalid - 5-yl) - 2- (trifluoromethyl) propanamid aus erfindungsgemäßen Pflastern mit 1% Wirkstoff in der Matrix**

| | 1 h | 4 h | 8 h | 24 h |
|---|---|---|---|---|
| 1. Prüfling | 39 % | 72 % | 80 % | 81 % |
| 2. Prüfling | 39 % | 68 % | 73 % | 74 % |
| 3. Prüfling | 40 % | 74 % | 91 % | 92 % |
| 4. Prüfling | 40 % | 73 % | 84 % | 85 % |
| 5. Prüfung | 39 % | 73 % | 85 % | 87 % |
| 6. Prüfling | 39 % | 71 % | 82 % | 84 % |
| Mittelwert | 39 % | 72 % | 83 % | 84 % |

Überraschenderweise setzen die erfindungsgemäßen Pflaster bereits nach 4h den weitaus größten Teil ihrer Beladung mit Wirkstoff frei und stellen diesen für eine Behandlung zur Verfügung. Die erfindungsgemäßen Pflaster sind daher geeignet, das beschriebene Problem der Überladung einer oralen Arzneiform mit PRASL zumindest deutlich zu reduzieren.

Durch die Reduktion der Einsatzenge an Wirkstoff pro Arzneiform, bieten die erfindungsgemäßen Pflaster einen wirtschaftlichen Vorteil im Vergleich zu einer oralen Arzneiform. Darüber hinaus wird durch die Reduktion der Wirkstoffmenge pro Arzneiform das Umweltrisiko, das durch die Arzneiform ausgeht, erheblich reduziert.

Die Fähigkeit der erfindungsgemäßen Pflaster, den Wirkstoff durch humane Haut zu transportieren, wurde mittels Permeationsstudien an exzidierter Humanhaut untersucht.Hierbei kam das etablierte Modell der Franz-Diffusionszelle zum Einsatz. Es wurden ebenfalls je 6 Pflaster untersucht, die mit jeweils 1% (R)-3-{1-[2-Fluor-5-(trifluormethyl) phenyl] cyclopropyl}-2-hydroxy -N-(phthalid - 5-yl) - 2- (trifluoromethyl) propanamid in der Matrix beladen waren.

**Tabelle 2 Kumulativer Flux von (R)-3-{1-[2-Fluor-5-(trifluormethyl) phenyll cyclopropyl}-2-hydroxy -N-(phthalid - 5-yl) - 2- (trifluoromethyl) propanamid in aus erfindungsgemäßen Pflastern mit 1% Wirkstoff in der Matrix durch Humanhaut.**

| | 0 h | 6 h | 12 h | 24 h | 30 h | 48 h |
|---|---|---|---|---|---|---|
| 1. Prüfling | n.w. | n.w. | n.w. | 3,60 [µg/cm²] | 4,86 [µg/cm²] | 8,96 [µg/cm²] |
| 2. Prüfling | n.w. | n.w. | n.w. | 3.70 [µg/cm²] | 5,26 [µg/cm²] | 10,40 [µg/cm²] |
| 3. Prüfling | n.w. | n.w. | n.w. | 3,17 [µg/cm²] | 4,36 [µg/cm²] | 8,20 [µg/cm²] |
| 4. Prüfling | n.w. | n.w. | 0,92 [µg/cm²] | 5,45 [µg/cm²] | 7,03 [µg/cm²] | 11,96 [µg/cm²] |
| 5. Prüfling | n.w. | n.w. | n.w. | 3,35 [µg/cm²] | 4,78 [µg/cm²] | 9,51 [µg/cm²] |
| 6. Prüfling | n.w. | n.w | n.w. | 2,94 [µg/cm²] | 3,97 [µg/cm²] | 7,33 [µg/cm²] |
| Mittelwert | w.w. | n.w. | 0,15 [µg/cm²] | 3,70 [µg/cm²] | 5,04 [µg/cm²] | 9,39 [µg/cm²] |

| | | | | | | |
|---|---|---|---|---|---|---|
| n.w. = unterhalb der Nachweisgrenze | | | | | | |

Überraschenderweise zeigen die Ergebnisse, dass bereits ein maximal 15cm² großes erfindungsgemäßes Pflaster, das frei von penetrationsverstärkenden Hilfsstoffen ist, in der Lage ist, therapeutisch relevante Mengen (30-50 µg/d) an (R)-3-{1-[2-Fluor-5-(trifluormethyl) phenyl] cycloprvpyl}-2-hydroxy -N-(phthalid - 5-yl) - 2- (trifluoromethyl) propanamid durch die Haut zu transportieren.

Durch die geringe Größe eines transdermalen Pflasters ist eine hohe Patientenakzeptanz erwarten lässt. Der Verzicht auf penetrationsverstärkende Zusätze senkt das Risiko des Auftretens von Wirkstoff-Hilfsstoff Inkompatibilitäten sowie von irritativen Hautreaktionen senkt und damit einen weiteren Vorteil der erfindungsgemäßen Pflaster darstellt.

Durch die gleichförmigen Flussraten des Wirkstoffes ermöglicht eine erfindungsgemeine Arzneiform darüber hinaus eine Reduktion des Dosierinterwalles von einer täglichen Anwendung der oralen Arzneiform hin zu einer wöchentlichen Anwendung.

Diese Reduktion stellt einen weiteren Vorteil der erfindungsgemäßen Arzneiform dar.

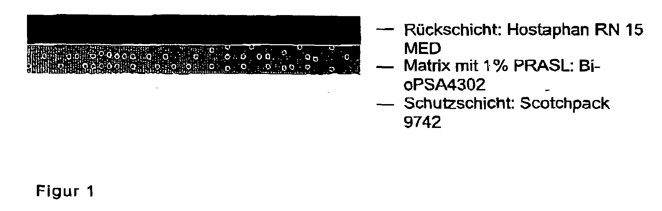

## Patentansprüche

1. Transdermales Pflaster, den Wirkstoff der allgemeinen Formel 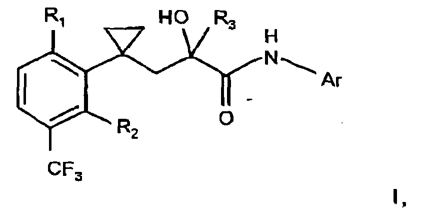 enthaltend , wobei R₁ und R₂ unabhängig voneinander H oder F, R₃ CH₃ oder CF₃ und AR 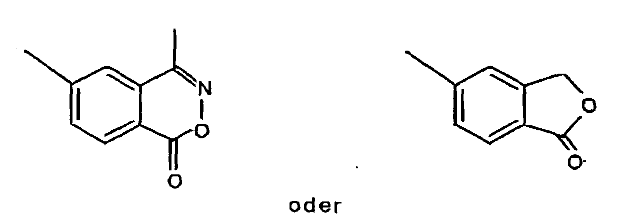 oder pharmazeutisch geeignete Derivate oder Abkömmlinge hiervon sind (Progesteron A-spezifische Liganden, PRASL),
**dadurch gekennzeichnet, dass** das transdermale Pflaster aus einer Rückschicht, mindestens einer daran haftenden, wirkstoffhaltigen Klebschicht auf Basis eines Silikonpolymers, eines Polyisobutylenpolymers (PIB), eines Polyacrylatpolymers oder eines Styrolblockcopolymers mit Butadien oder Isopren (SBS oder SIS), wobei der Wirkstoff in einer Konzentration von 0,1-10%, bezogen auf das Gesamtgewicht der Klebermatrix enthalten ist, sowie eines entfernbaren Schutzfilmes besteht.

2. Transdermales Pflaster nach Anspruch 1,
**dadurch gekennzeichnet, dass** der Wirkstoff in einer Konzentration von 0,1-5%, bezogen auf das Gesamtgewicht der Klebermatrix, vorzugsweise in der Konzentrationen von 0,1-2% bezogen auf das Gesamtgewicht der Klebermatrix enthalten ist.

3. Transdermales Pflaster nach den Ansprüchen 1 und 2,
**dadurch gekennzeichnet, dass** die Löslichkeit des Wirkstoffes in der Klebschicht von 0,1-5% , vorzugsweise von 0,5-2% definiert ist.

4. Transdermales Pflaster nach einem oder mehreren der Ansprüche 1 bis 3,
**dadurch gekennzeichnet, dass** der Wirkstoff in weniger als 50 % ungelöst in der Matrix eingebettet ist.

5. Transdermales Pflaster nach Anspruch 4,
**dadurch gekennzeichnet, dass** der Wirkstoff in der Form von Mikropartikel und Mikrotropfen, vorzugsweise als Nanopartikel oder Nanotropfen gleichmäßig dispergiert in der Matrix vorliegt.

6. Transdermales Pflaster nach den Ansprüchen 4 und 5,
**dadurch gekennzeichnet, dass** der Wirkstoff in amorpher Form in der Matrix vorliegt.

7. Transdermales Pflaster nach einem oder mehreren der Ansprüche 1 bis 6,
**dadurch gekennzeichnet, dass** die wirkstoffhaltige Matrix einen Kristallisationsinhibitor enthält, der aus der Gruppe ausgewählt ist, die N-Vinyllactam-Polymere wie N-Vinyl-1-Azacycloheptan-2-on-Homapolymer und N-Vinyl-piperidin-2-on-Homopolymer und insbesondere Polymere des Vinylpyrrolidons, wie Polyvidon (Kollidon TM), oder Co-Polymere des Vinylpyrrolidons mit Vinylacetat (Copovidone) umfasst.

8. Transdermales Pflaster nach einem oder mehreren der Ansprüche 1 bis 7,
**dadurch gekennzeichnet, dass** die wirkstoffhaltige Matrix als Kristallisationsinhibitor ein Copovidon aus 6 Teilen Vinylpyrrolidon und 4 Teilen Vinylacetat (Kollidon TM VA 64) enthält.

9. Transdermales Pflaster nach einem oder mehreren der Ansprüche 1 bis 8,
**dadurch gekennzeichnet, dass** die Klebschicht einen Klebestoff auf Silikonbasis, enthält, der sich durch einen hohen Anteil an Polymer gegenüber dem Harz auszeichnen, vorzugsweise aminkompatible Klebstoffe mit einem Masseverhältnis Polymer zu Harz größer oder gleich 40% zu 60%.

10. Transdermales Pflaster nach einem oder mehreren der Ansprüche 1 bis 8,
**dadurch gekennzeichnet, dass** die Klebschicht Klebestoffe auf Polyisobutylenbasis enthält.

11. Transdermales Pflaster nach einem oder mehreren der Ansprüche 1 bis 10.
**dadurch gekennzeichnet, dass** es auch ein Östrogen enthält, das aus der Gruppe ausgewählt ist, die 17-bota-Estradiol, Ethinylestradiol, Estradiolvalerat, Estradiolcypionat, Estradiolacetat und Estradiolbenzoat umfasst.

12. Transdermales Pflaster nach einem oder mehreren der Ansprüche 1 bis 11.
**dadurch gekennzeichnet, dass** es innerhalb eines 7-tägigen Anwendungszyklus mehr als 30%, vorzugsweise mehr als 50% seiner Beladung mit dem in Anspruch 1 definiertem Wirkstoff freisetzt.

13. Verfahren zur Herstellung des transdermalen Pflasters nach einem oder mehreren der Ansprüche 1-12.
**dadurch gekennzeichnet, dass** der entsprechende Wirkstoff in einer Kombination aus mindestens zwei Prozessiösemittein aufgenommen wird, von denen eines ein geringes Lösungsvermögen für den Wirkstoff und ein weiteres ein hohes Lösungsvermögen für den Wirkstoff besitzt, wobei letzteres nach Vermengung mit der Klebermatrix durch Trocknungsprozesse zuerst aus dem Ansatz entfernt wird

14. Verfahren zur Herstellung des transdermalen Pflasters nach Anspruch 13,
**dadurch gekennzeichnet, dass** das Lösungsmittel mit geringem Lösungsvermögen für den Wirkstoff 1,4 Dioxan und das Lösungsmittel mit hohem Lösungsvermögen Heptan ist.
